# EUROPEAN PATENT SPECIFICATION

(11) **EP 1 232 059 B1**
(45) Date of publication and mention of the grant of the patent: **25.10.2006**
(21) Application number: 01966559.5
(22) Date of filing: 04.09.2001
(51) Int. Cl.: B32B 27/12, D04H 1/60

(54) **COMPOSITE SHEET MATERIAL**
BAHNFÖRMIGES VERBUNDMATERIAL
MATERIAU COMPOSITE EN FEUILLE

(30) Priority: 01.09.2000 US 229945 P; 30.08.2001 US 942405
(43) Date of publication of application: 21.08.2002
(73) Proprietor: E. I. du Pont de Nemours and Company, Wilmington, Delaware 19898 (US)
(72) Inventor: HUFFINES, Prentice, Lee, Old Hickory, TN 37138 (US); LIM, Hyun, Sung, Midlothian, VA 23113 (US); MARTIN, Oscar, L., Jr., Hermitage, TN 37076 (US); SUH, Hageun, Nashville, TN 37215 (US)
(74) Representative: Woodman, Derek
(86) International application number: PCT/US2001/027419
(87) International publication number: WO 2002/018137

(56) References cited:
- WO-A-00/73058
- WO-A-97/45259

## Description

### FIELD OF THE INVENTION

This invention relates to a moisture vapor permeable, substantially liquid impermeable composite sheet structure useful in medical garments. More particularly, the invention is directed to a composite laminated sheet comprising a thin extruded moisture vapor permeable film sandwiched between two nonwoven webs, one of which is a powder-bonded nonwoven web, the other of which is a non powder-bonded nonwoven web. The composite laminated sheet acts as a barrier to liquids, bacteria, viruses and odors, and yet is also highly permeable to moisture vapor.

### BACKGROUND OF THE INVENTION

The use of laminates of films and nonwovens for medical applications, including medical garments, is known in the art. For example, Shehata, U.S. Patent 5,445,874 describes laminates that are waterproof, blood-proof, and virus proof with a high moisture vapor transmission rate. The laminates include a solid extruded film layer of a polymer comprising 60-70% butylene terephthalate units and the balance a polyester glycol sandwiched between two woven or nonwoven fabrics. The thickness of the film layer is typically between about 0.75 to about 3 mil (19 to 76 micrometers) and can be extruded directly onto one of the fabric layers. Alternately, the film layer can be extruded onto a release layer or can be extruded as a blown film. The films can be laminated onto the fabric by powder lamination, hot melt lamination, and wet adhesive lamination. The laminates are suitable for use in re-usable clothing or apparel where it is desirable or necessary to prevent liquids from penetrating through to the wearer's skin. Garments made from the laminates are washable and sterilizable a minimum of 100 times.

PCT Publication No. WO 97/45259 to Carroll et al. discloses a breathable composite sheet material comprised of a moisture vapor permeable thermoplastic film adhered to a fibrous substrate. The breathable thermo-plastic film is primarily comprised of a thermoplastic polymeric material selected from the group of block copolyether esters, block copolyether amides and polyurethanes. The fibrous substrate is a nonwoven sheet made primarily of polymeric fibers that are incompatible with the film, such as a polyolefin fibers. The film is adhered to the fibrous substrate by extruding a layer of the molten film-forming polymer directly onto the fibrous substrate and then mechanically engaging the film and the fibers of the substrate, as for example by pressing the molten film into the fibrous substrate in a nip formed between two rolls.

WO 00/73058 discloses a moisture vapor permeable, substantially liquid impermeable composite sheet material including a powder-bonded nonwoven web adhered to a moisture vapor permeable thermoplastic film. The nonwoven web includes a first layer comprised primarily of fibers, some of which are compatible with, and some of which are incompatible with the bonding adhesive and the thermoplastic film. A method of making the composite sheet material and an item of apparel made from the sheet material are also provided.

Adhesive lamination, thermal lamination and extrusion coating methods have all been used to produce composite sheets of a fibrous nonwoven substrate and a moisture vapor permeable, substantially liquid impermeable film. It has been possible to make such composite sheets with good barrier properties so long as the moisture vapor permeable film is relatively thick (i.e., greater than about 25 micrometers). However, it has been difficult to make such composite sheets with thinner films without sacrificing important barrier properties. Very thin moisture vapor permeable films are desirable in a composite sheet because thinner films facilitate greater flux of moisture vapor through the composite sheet and because thinner films use less of the film material and are accordingly less expensive to produce.

Adhesive lamination is carried out in a post film formation step. For adhesive lamination to be feasible, the moisture vapor permeable film must have enough tensile strength and tear strength so that the film can be formed, wound onto a roll, and later unwound and handled during the adhesive lamination process. It is difficult to handle moisture vapor permeable films less than about 1 mil (25 micrometers) in thickness during the adhesive lamination process without tearing the film or introducing defects into the film.

Thermal lamination of moisture vapor permeable films less than 25 micrometers thick has resulted in composite sheet materials with inadequate barrier properties. When composite sheets are made by thermally laminating a thin film to a fibrous substrate, the thin film handling problems associated with adhesive lamination, as described above, are encountered. In addition, to carry out a thermal lamination, the film must be subjected to elevated temperatures and pressures so as to soften the film and force it into mechanical engagement with the fibrous substrate. Generally, the bond strength between the film and the fibrous substrate increases with increasing lamination temperatures and increasing nip pressures. Unfortunately, when moisture vapor permeable films with a thickness of less than 25 micrometers are subjected to the increased temperatures and pressures needed to obtain adequate bond strength between the layers in the composite sheet, small holes can develop in the film such that the composite sheet does not exhibit the barrier properties desired in a composite sheet for use in medical apparel.

It is the practice in many hospitals to use surgical gowns only once, and to discard the gowns thereafter. There remains a need for breathable sheet materials having a high barrier level to viral penetration and which also can be prepared at low cost for use in single-use disposable medical garments. An ideal material must also be strong enough so that it does not rip or delaminate under normal use regardless of whether the material is dry or wet. Where the sheet material is to be used in apparel, it is also important that the material be flexible, soft and drapable. In addition, where the sheet material is to be used in medical apparel, it is important that the sheet not generate fiber lint that might contaminate a medical environment.

### SUMMARY OF THE INVENTION

One embodiment of the present invention is a moisture vapor permeable, substantially liquid impermeable composite sheet material having a hydrohead measured according to ISO-811 of at least 50 cm, and a moisture vapor permeability of at least 600g/m²/24 hr, comprising:
a moisture vapor permeable non-porous polymeric film having a first side and a second side,
a first nonwoven layer comprising a moisture vapor permeable powder-bonded nonwoven layer, said powder-bonded layer comprising a nonwoven web of fibers, wherein greater than 95 weight percent of the fibers in the nonwoven web are compatible with said polymeric film, said first nonwoven layer being adhered to the first side of the polymeric film by extrusion of said film onto said first nonwoven layer, and a second moisture vapor permeable nonwoven layer adhered to the second side of the film, wherein said second nonwoven layer is not a powder-bonded layer.

Another embodiment of the present invention is directed to a garment or other protective cover prepared from such a moisture vapor permeable, substantially liquid impermeable composite sheet.

### BRIEF DESCRIPTION OF THE DRAWINGS

The accompanying drawings, which are incorporated in and constitute a part of this specification, illustrate the presently preferred embodiments of the invention and, together with the description, serve to explain the principles of the invention.
Figure 1 is a cross-sectional view of the composite sheet structure of the invention.
Figure 2 is a schematic representation of a process by which the powder-bonded nonwoven layer of the composite sheet of the invention is extrusion coated with the film layer.
Figure 3 is a schematic representation of an alternate process in which a bilayer film is co-extruded onto the powder-bonded nonwoven layer.

### DETAILED DESCRIPTION

The term "compatibility" as used herein, refers to the degree to which polymer materials are miscible with each other and/or interact with each other. "Incompatible" materials, as used herein, means materials that are substantially immiscible with each other or do not interact with each other. Incompatible materials do not wet or adhere well to each other, even when heated. As used herein, "compatible" materials are materials that are not "incompatible" with each other, as defined above. For purposes of this application, a fiber is deemed to be compatible with a synthetic adhesive or with another polymer if the adhesive or other polymer is miscible with material that comprises the majority of the fiber and if the adhesive or other polymer readily wets the fiber or if the adhesive or polymer can adhere well to the fiber.

The term "nonwoven fabric, sheet, layer, or web" as used herein means a structure of individual fibers or filaments that are positioned in a random manner to form a planar material without an identifiable pattern by means other than knitting or weaving. As used herein, the term "fiber" means an elongated strand of defined length, such as staple fibers formed by cutting a continuous strand into lengths and the term "filament" means a generally continuous strand that has a very large ratio of length to diameter.

The terms "powder-bonded nonwoven web and powder-bonded nonwoven layer" as used herein refer to a bonded nonwoven fabric formed by depositing a powder adhesive onto an unbonded fibrous web, such as a carded web, in a way that the powder adhesive is distributed throughout the thickness of the fibrous web. A powder adhesive is selected which melts at a temperature below the melting point of the fibers in the fibrous web. Thereafter, the powder-containing web is heated to melt the powder adhesive without melting the fibers of the fibrous web to form a powder-bonded nonwoven web. Powder-bonded nonwoven fabrics are distinguished from sheets made using powder lamination methods such as those described in Shehata U.S. Patent No. 5,445,874 where an activated web consisting of an adhesive material is formed from an adhesive powder. In powder lamination, the powder adhesive bonds two substrates together, such as a woven or nonwoven substrate with a film layer and the adhesive powder is concentrated primarily at the interface between the two substrates. In powder lamination, the powder adhesive does not contribute to any significant degree to the bonding within the nonwoven substrate.

The term "spunbond" as used herein means a nonwoven sheet formed by melt extrusion of a polymer into strands that are quenched and drawn, usually by high velocity air, to strengthen the filaments. The filaments are collected on a forming surface and bonded, often by application of heat and pressure using a patterned bonding roll.

As used herein, the "machine direction" is the long direction within the plane of a sheet, i.e., the direction in which the sheet is produced. The "cross direction" is the direction within the plane of the sheet that is perpendicular to the machine direction.

As used herein, the term "laminate" means a composite material made from two or more layers or webs of material which have been bonded or otherwise attached to one another.

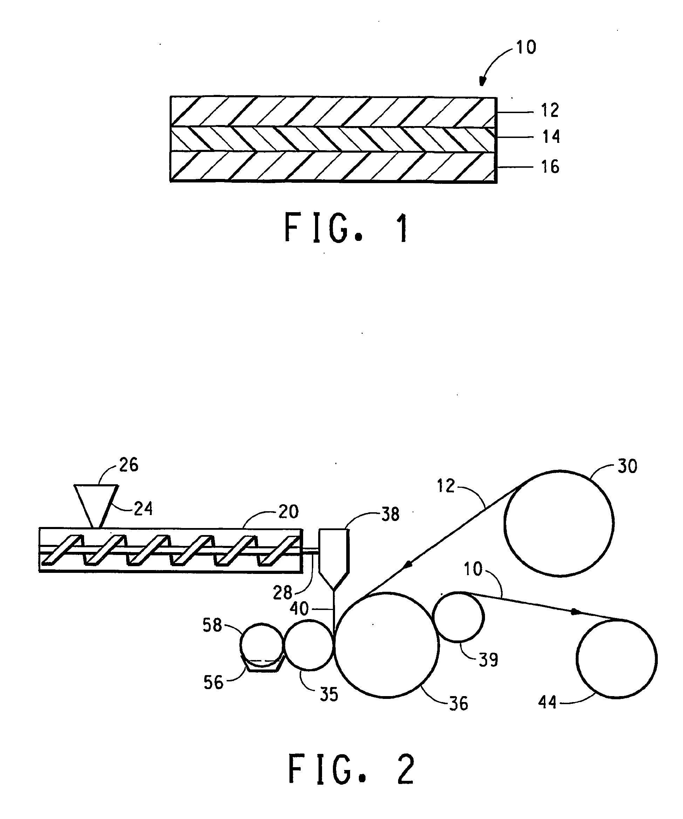

Referring to Figure 1, the composite laminated sheet **10** of the current invention is shown. Composite laminated sheet **10** comprises a moisture vapor permeable powder-bonded nonwoven layer **12** onto which a substantially liquid impermeable, moisture vapor permeable polymer film **14** is extrusion coated. Film **14** can be a single layer or multi-layer film. A second moisture vapor permeable nonwoven layer **16** is adhered to the film layer on the side of the film layer opposite that which is adhered to the powder-bonded nonwoven layer.

The powder-bonded nonwoven layer **12** used in the composite laminated sheet of the invention is prepared using methods known in the art, such as the process described in Zimmerman et al. U.S. Patent 4,845,583. A carded web of staple fibers is optionally passed through a web spreading section prior to applying a powdered adhesive material. Preferably the staple fibers of the carded web have a length between about 1 and 2 inches (2.54 to 5.08 cm) and a denier between 1 and 2 denier per filament (1.1 to 2.2 dtex). An adhesive powder is applied to the carded web using a powder-depositing device. The powder drops onto the web and is distributed through the web by gravity. Excess powder falls through the web and is collected for recycling. The weight of powder deposited in the carded web is preferably from about 8 to about 30 weight percent, and more preferably between about 15 to 25 weight percent based on the total weight of the carded web and adhesive powder. Bonding of the nonwoven layer can be achieved by applying heat to the powder-infiltrated web. For example, the web can be passed through an oven, such as an infrared oven in which the adhesive powder fuses and bonds the fibers of the web at fiber crossover points where the fibers and the bonding material come into contact. Upon leaving the oven, the web is subjected to light pressure by means of a nip roll. The powder-bonded nonwoven layer preferably has a basis weight in the range of 13.8 g/m² to 60 g/m², more preferably in the range of 16 g/m² to 34.5 g/m², and most preferably 16 g/m² to 29.6 g/m².

Powder-bonded nonwoven layer **12** is preferably prepared from a carded web of staple fibers, in which the fibers are comprised of a synthetic polymer that is compatible with both the polymer of film layer **14** and the adhesive powder used bond the staple fibers in the powder-bonded nonwoven layer. Greater than 95 weight percent of the staple fibers in the carded web based on the total weight of staple fibers in the carded web comprise fibers which are compatible with the adhesive powder and the film layer. As the level of incompatible staple fibers in the carded web is raised above about 10 weight percent, the degree of bonding in the powder-bonded nonwoven layer is reduced which can result in a powder-bonded nonwoven layer with reduced strength which can cause handling problems as well as increased linting. In end uses such as medical garments and drapes, linting is especially undesirable.

The fibrous components of the powder-bonded nonwoven layer preferably comprise a polyester such as poly(ethylene terephthalate), poly(1,3-propylene terephthalate) and copolymers thereof. Such polyester polymers are compatible with block polyether copolymers such as a block polyether ester copolymers, with polyurethane copolymers, with poly(etherimide) ester copolymers, and with combinations thereof. One type of polyester fiber that can be used are shaped polyester fibers, such as shaped polyester fibers with a scalloped-oval cross-section as disclosed in U.S. Patent 3,914,488 to Garrafa (assigned to DuPont). It is believed that where the polyester fibers comprise at least 10% of such shaped fibers, channels are created in the fibrous substrate through which moisture vapor can be more efficiently conveyed through the composite laminated sheet. Alternately, a laminate of a powder-bonded polyamide nonwoven and a poly(etheramide) film can be used.

The powder adhesive that is used in the powder-bonded nonwoven layer is comprised of a thermoplastic polymer that melts at a temperature below the melting point of the staple fibers used in the carded web. A powder adhesive is selected which is compatible with the film layer as well as with greater than 95 weight percent of the staple fiber components of the carded web so as to provide good bonding both within the powder-bonded nonwoven layer as well as between the powder-bonded nonwoven layer and the film layer which is extruded thereon.

In a preferred embodiment, the powder-bonded nonwoven layer is prepared from a carded web of polyester staple fibers and a polyester copolymer adhesive powder. Suitable polyester copolymer adhesive powders include polyester copolymer powders such as those available from EMS-American Grilon, Inc. Typical copolyester adhesives have melting points of from 100 to 130 °C. and are available as coarse powders (200-420 micrometers or 70-40 U.S. standard mesh), medium powders (80-200 micrometers or 200-70 U.S. standard mesh) and fine powders (80 micrometers or less, or finer than 200 U.S. standard mesh), the medium powders being preferred when mechanical applicators are used to apply the adhesive powder to the web.

Film layer **14** comprises a polymeric material that can be extruded as a thin, continuous, moisture vapor permeable, and substantially liquid impermeable film. The film layer is extruded directly onto the powder-bonded nonwoven layer and adheres to both the fibrous and adhesive components of the powder-bonded web. Preferably, the extruded film is less than about 1 mil (25 micrometers) thick, more preferably less than about 0.75 mil (19 micrometers) thick, and most preferably less than about 0.60 mil (15.2 micrometers) thick. The film layer **14** is preferably comprised of a block polyether copolymer such as a block polyether ester copolymer, a polyetheramide copolymer, a polyurethane copolymer, a poly(etherimide) ester copolymer, polyvinyl alcohols, or a combination thereof. Preferred copolyether ester block copolymers are segmented elastomers having soft polyether segments and hard polyester segments, as disclosed in Hagman, U.S. Patent No. 4,739,012. Suitable copolyether ester block copolymers are sold by DuPont under the name Hytrel®. Hytrel® is a registered trademark of DuPont. Suitable copolyether amide polymers are copolyamides available under the name Pebax® from Atochem Inc. of Glen Rock, New Jersey, USA. Pebax® is a registered trademark of Elf Atochem, S.A. of Paris, France. Suitable polyurethanes are thermoplastic urethanes available under the name Estane® from The B.F. Goodrich Company of Cleveland, Ohio, USA. Suitable copoly(etherimide) esters are described in Hoeschele et al., U.S. Patent 4,868,062.

A preferred film comprises a layer of nonporous (monolithic) copolyether ester copolymer. Monolithic copolyether ester membranes such as those made of Hytrel® copolyether ester elastomer, sold by DuPont, absorb water from the "wet" side of the membrane into the polymer matrix and release the water from the "dry" side, a process which has been referred to in the art as pervaporation. The rate at which water vapor is released on the dry side of a Hytrel® film depends on the amount of water that has diffused into the membrane, which in turn depends on the relative humidity on the wet side of the membrane. At very high relative humidity, or when liquid water is in contact with the membrane on the wet side, the water vapor transmission rate of a Hytrel® membrane can be extremely high.

The film layer **14** is preferably applied to the powder-bonded nonwoven layer by extrusion coating. In an extrusion coating process, a uniform (monolithic) molten extrudate is coated onto the powder-bonded nonwoven layer. The molten polymer and the powder-bonded web are brought into intimate contact as the molten polymer cools and bonds with the web. Such contact and bonding can be enhanced by passing the layers through a nip formed between two rolls. Alternatively, the molten polymer can be pulled into contact with the powder-bonded nonwoven web by passing the coated web over a suction inlet such that a vacuum pulls the molten polymer into contact with the web as the polymer cools and bonds with the web. During the extrusion coating process, some or all of the powder adhesive in the powder-bonded nonwoven web may melt, which is believed to provide improved bonding between the thin film layer and the powder-bonded nonwoven web. The bonding between the polymeric film layer and the adhesive present in the powder-bonded nonwoven web is believed to make it easier to produce a very thin moisture vapor permeable film that is substantially free of pinholes or other defects, yet which has a relatively high rate of moisture vapor transmission. As used herein, "pinholes" means small holes inadvertently formed in a film either during manufacture or processing of the film.

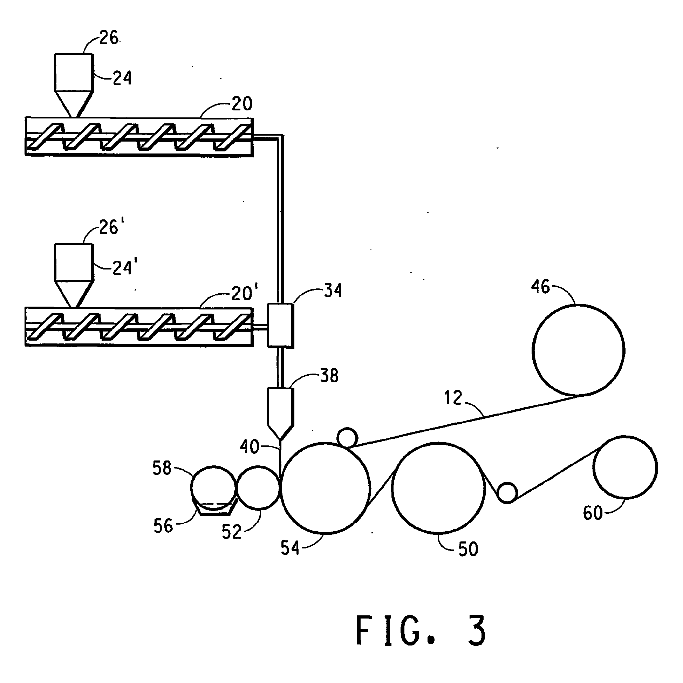

One preferred means for applying the film layer to the powder-bonded nonwoven layer is illustrated in Figure 2. Melt-processable polymer is fed in pellet form, along with any additives, into an inlet **26** of an extruder hopper **24,** preferably under a nitrogen purge. The polymer is melted and mixed in a screw extruder **20** at a screw speed in the range of 100 to 200 rpm, depending on the dimensions of the extruder and the properties of the polymer. The melted mixture is discharged from the extruder under pressure through a heated line **28** to a flat film die **38.** The polymer is discharged from the flat film die **38** at a temperature above the melting temperature of the polymer, and preferably at a temperature in the range of 180°C to 240 °C. The polymer melt **40** discharging from the flat film die **38** coats the powder-bonded nonwoven layer **12** which is provided from supply roll **30.**

Preferably, the powder-bonded nonwoven web **12** passes under the die at a speed that is coordinated with the speed of the extruder so as to obtain a very thin film that preferably has a thickness of less than 25 micrometers. The coated powder-bonded nonwoven web enters a nip formed between nip roll **35** and roll **36,** which rolls are maintained at a temperature selected to obtain a composite laminated sheet with a desired bond strength and moisture vapor permeability. The temperature of rolls **35** and **36** is preferably within the range of 10 °C to 120 °C. Higher roll temperatures yield a composite sheet having higher bond strength, while lower roll temperatures yield composite sheets with higher moisture vapor permeability. Preferably, nip roll **35** is a smooth rubber roll with a low-stick surface coating while roll **36** is a metal roll. Nip roll **35** can also have a matte or textured finish to prevent sticking of the film layer. A textured embossing roll may be used in place of the metal roll for the roll **36** if a composite laminated sheet with a more textured film layer is desired. Passing the coated web through the nip formed between cooler rolls **35** and **36** quenches the polymer melt while at the same time pressing the polymer melt **40** into contact with the fibers and adhesive of the powder-bonded nonwoven layer **12.** A water dip pan **56** with associated roll **58** can be used to increase the quench rate and to prevent sticking. The nip pressure applied should be sufficient to achieve the desired degree of bonding between the film and the nonwoven layer but not so great as to create pinholes in the film layer. The powder-bonded nonwoven/film laminate **10** is transferred from the roll **36** to another smaller roll **39** before being wound up on a collection roll **44.** The coated composite laminated sheet **10** is transferred from the roll **36** to another smaller roll **39** before being wound up on a collection roll **44.**

The second nonwoven layer **16** (Fig. 1) can be fed into the nip on the side of the film layer opposite the side to which the powder-bonded nonwoven layer **12** is adhered. When the second nonwoven layer is incorporated into the composite laminated sheet in this manner, the side of the second nonwoven layer contacting the film layer will generally be adhered to the film layer over substantially the entire surface thereof. This can result in a composite laminated sheet that is stiffer than desired for medical garments or other apparel end uses due to the high degree of bonding between the film layer and both the powder-bonded nonwoven layer and the second nonwoven layer. If the second nonwoven layer is made of polymeric fibers or filaments which are incompatible with the film layer, the bond strength between the film and the second nonwoven layer may be so low as to cause the second nonwoven to delaminate from the film layer during use. Preferably the bond strength between the film and the second nonwoven layer is at least 19.7 grams/cm (50 grams/inch) when the composite sheet material is either wet or dry, more preferably between 19.7 and 118.2 grams/cm (50 and 300 grams/inch).

The film layer **14** (Fig. 1) of the composite laminated sheet can be comprised of multiple layers. Such a film may be co-extruded with layers comprised of one or more of the above-described breathable thermoplastic film materials. Examples of such multiple layer moisture vapor permeable films, which typically comprise a comparatively more hydrophobic elastomer layer and a comparatively more hydrophilic elastomer layer, are disclosed in Ostapchenko, U.S. Patent No. 4,725,481. In a preferred embodiment, a multi-layer film (in a bi-layer execution) is extruded onto the powder-bonded nonwoven layer **12** with the comparatively more hydrophobic elastomer layer facing outwardly from the powder-bonded web and the comparatively more hydrophilic elastomer layer bonded to the powder-bonded nonwoven layer. Typically, for a given thickness, the hydrophobic elastomer layer exhibits a lower moisture vapor transmission rate than the hydrophilic elastomer layer due to its comparatively lower moisture content under in-use conditions. However, when employed in a comparatively thin layer, the effect of the hydrophobic lower moisture content film layer does not significantly diminish the moisture vapor transmission rate of the overall composite laminated sheet. Preferably, the comparatively more hydrophobic elastomer comprises between 20 and 30 percent of the total thickness of the composite film layer. Medical garments are preferably manufactured from the composite laminated sheet of the invention with the second nonwoven layer facing outwardly, away from the person wearing the garment. The comparatively more hydrophobic layer, which is bonded to the second nonwoven layer, swells less and results in less puckering of the fabric when the outer surface of the garment is contacted with aqueous materials compared to a garment that is made with the second nonwoven layer facing towards the person wearing the garment. Because the majority of the film layer is comprised of the comparatively more hydrophilic layer, the garment also maintains an excellent moisture vapor transmission rate to ensure the comfort of the wearer.

Figure 3 illustrates a process for extrusion coating of a two-layer film onto a powder-bonded nonwoven layer. A first melt-processable polymer is fed in pellet form, along with any additives, into the inlet **26** of extruder hopper **24,** while a second melt-processable polymer is fed in pellet form, along with any additives, into the inlet **26'** of extruder hopper **24'.** The polymer is melted and mixed in the screw extruders **20** and **20'** at screw speeds that depend on the dimensions of the extruders and the properties of the polymer. The melted mixture is discharged from the extruder under pressure through heated lines to a melt combining block **34,** where a multiple layer melt is formed that is extruded as a multiple layer film through flat film die **38.** The polymer is discharged from the flat film die **38** at a temperature above the melting point of the polymer mixture, and preferably at a temperature in the range of 180°C to 240 °C. The polymer melt **40** discharging from the flat film die **38** coats the powder-bonded nonwoven layer **12** provided from a supply roll **46.** Preferably, the powder-bonded nonwoven layer **12** passes under the die 38 at a speed that is coordinated with the speed of the extruder so as to obtain a film thickness of less than 25 micrometers, preferably less than 19 micrometers, on the powder-bonded nonwoven layer. The coated web enters a nip formed between nip roll 52 and roll **54,** which rolls are maintained at a temperature selected to obtain a composite laminated sheet with a desired bond strength and moisture vapor permeability. A water dip pan **56** with associated roll **58** can be used to increase the quench rate and to prevent sticking. Alternatively, a water mist applied to the film layer or a water bath associated with roll **52** may be used. An optional cooled quench roll **50** can be used to provide additional cooling prior to winding the composite laminated sheet product on collection roll 60.

The second nonwoven layer **16** (Fig. 1) is preferably formed of fibers or filaments of a melt-processable polymer such as polypropylene, polyethylene, or polyester, that can be heat-sealed to form seams in medical garments. Examples of nonwoven fabrics suitable as the second nonwoven layer include spunbond, hydroentangled, stitchbonded, and flash-spun plexifilamentary nonwoven fabrics. For example, Sontara® hydroentangled polyester fabrics or Tyvek® flash-spun polyethylene sheets, available from DuPont, can be used as the second nonwoven layer in the composite laminated sheet of the invention. If a flash-spun sheet is used as the second nonwoven layer, it preferably has been point-bonded and softened to provide the desired hand and drapability. Spunbond polypropylene or polyester nonwoven fabrics are preferred. The second nonwoven layer preferably has a basis weight in the range of about 0.5 oz/yd² to 2 oz/yd² (17 g/m² to 68 g/m²), more preferably in the range of about 0.8 oz/yd² to 1.2 oz/yd² (27 g/m² to 40.7 g/m²). Thicker layers generally result in stronger seam strength when medical garments are fabricated with heat-sealed seams with the second nonwoven layer forming the outer surface of the garment.

In a preferred embodiment, the second nonwoven layer is a polypropylene nonwoven fabric, such as a polypropylene spunbond fabric. Polypropylene melts at a lower temperature than polyesters such as poly(ethylene terephthalate), thus requiring lower temperatures for heat-sealing the seams of the medical garment. Heat-sealed seams are generally formed by overlaying two of the composite laminated sheets with what will be the outer surface of the garment (e.g. spunbond polypropylene) facing each other. When a garment is formed with the second nonwoven layer forming the outer surface of the garment, a seam is formed by contacting the edge of the overlaid sheets with a heated bar which fuses both of the second nonwoven layers to each other, thus forming a seam having a width approximately the same as the width of the heated bar. Lower heat-sealing temperatures are preferred since the film layer of the composite laminated sheet is less likely to melt or degrade during the heat-sealing process. It has also been found that when the second nonwoven layer is a polypropylene nonwoven fabric, higher seam strength and higher hydrostatic head measured at the seam are obtained compared to polyester nonwovens.

The second nonwoven layer can be adhered to the extrusion-coated powder-bonded nonwoven web using conventional adhesive lamination methods including powder lamination, hot melt lamination, and wet adhesive lamination. The lamination is preferably done in a way that provides a composite laminated sheet having the desired softness and drapability, and a moisture vapor transmission rate of at least 600 g/m²/24 hr, preferably at least 1000 g/m²/24 hr. In a preferred embodiment, the second nonwoven layer is joined to the film side of the extrusion-coated powder-bonded nonwoven web using an adhesive that has been applied in discrete areas such that the two components are bonded intermittently to each other. For example, the adhesive can be applied in a pattern or as randomly oriented filaments. The adhesive is preferably applied at about 2 g/m² to 4 g/m². If the adhesive loading is too high, the composite laminated sheet will be stiffer than desired. If it is too low, the bond strength between the film and the second nonwoven layer will be too low. Preferably the bond strength between the film and the second nonwoven layer is at least 19.7 grams/cm (50 grams/inch) when the composite sheet material is either wet or dry, more preferably between 19.7 and 118.2 grams/cm (50 grams/inch and 300 grams/inch).

The extrusion coated powder-bonded nonwoven web is generally less drapable and more noisy than is desired for use in garments. This is believed to be due to the high degree of bonding between the film layer and the powder-bonded nonwoven layer due to the high degree of compatibility between these layers. Surprisingly, it has been found that when the second nonwoven layer is adhered intermittently to the side of the film layer opposite that to which the powder-bonded nonwoven layer is adhered, the drapability of the composite laminated sheet of the current invention is significantly improved and the noisiness of the composite laminated sheet is significantly diminished, compared to that of the extrusion-coated powder-bonded web.

When used for garment end uses such as medical gowns, the composite laminated sheet **10** preferably has a basis weight between about 1.2 and 3 oz/yd² (41 to 102 g/m²) and more preferably between about 1.8 and 2.5 oz/yd² (61 to 85 g/m²), a grab tensile strength of at least 11 lb/inch (1925 N/m), and more preferably at least 15 Ib/inch (2625 N/m) in both the machine and cross directions, a hydrohead of at least 50 cm, and a moisture vapor permeability of at least 600 g/m²/24 hr, preferably at least 1000 g/m²/24hr. The composite laminated sheet of the invention also provides a viral barrier, with no viral penetration detected in 24 hours when measured according to ASTM F1671. The bond strength between the film and each of the nonwoven layers of the composite sheet is preferably at least 0.1 Ib/in (0.18 N/cm), and more preferably greater than about 0.15 Ib/in (0.26 N/cm).

When the composite laminated sheet is intended for use in medical garments, the fibrous components of the powder-bonded nonwoven layer and the second nonwoven layer are preferably selected so as to have some degree of hydrophobicity. Fibers or filaments having hydrophilic finishes applied thereto are generally less preferred. Hydrophilic fibers or filaments can contribute to soaking of the nonwoven layers by fluids, such as blood, by capillary action when the fluid contacts the edge of the fabric, such as may occur with the sleeve of a medical garment. Very fine fibers or filaments (low dtex per filament) have also been found to contribute to this problem. Preferably the fibrous components of nonwoven webs are no less than about 1 denier per filament (1.1 dtex), and more preferably no less than 1.5 denier per filament (1.65 dtex). Medical garments are preferably fabricated from the composite laminated sheet of the invention with the second nonwoven layer facing outwardly, away from the person wearing the garment. The second nonwoven layer is preferably treated with a repellant composition such as a fluorochemical treatment so that the second nonwoven layer will more readily repel mixtures of alcohol and water.

As shown in the examples below, the use of a powder-bonded polyester nonwoven web as a supporting substrate for the extruded film layer instead of nonwoven fabrics that are conventionally used in disposable medical garments, surprisingly allows thinner films and a lower overall basis weight to be used in the composite laminated sheet of the invention while providing superior viral barrier.

### EXAMPLES

In the description above and in the non-limiting examples that follow, the following test methods were employed to determine various reported characteristics and properties. ASTM refers to the American Society for Testing and Materials, TAPPI refers to the Technical Association of Pulp and Paper Industry, ISO refers to the International Organization for Standardization and INDA refers to Association of the Nonwoven Fabrics Industry.

Basis weight was determined by ASTM D-3776, which is hereby incorporated by reference, and is reported in g/m².

Denier was determined by ANSI-ASTM D 1577-73, which measures weight in gram per 9000 meter in length of yarn.

Tensile strength was determined by ASTM D 5035-95, which is hereby incorporated by reference, with the following modifications. In the test a 2.54 cm by 20.32 cm (1 inch by 8 inch) sample was clamped at opposite ends of the sample. The clamps were attached 12.7 cm (5 in) from each other on the sample. The sample was pulled steadily at a speed of 5.08 cm/min (2 in/min) until the sample broke. The force at break was recorded in pounds/inch and converted to Newtons/cm as the breaking tensile strength.

Film thickness was determined by ASTM Method D177-64, which is hereby incorporated by reference, and is reported in micrometers.

Grab Tensile Strength was determined by ASTM D 5034-95, which is hereby incorporated by reference, was measured in pounds/inch and is reported in Newtons/cm.

Elongation to Break of a sheet is a measure of the amount a sheet stretches prior to failure (breaking) in a strip tensile test. A 1.0 inch (2.54 cm) wide sample is mounted in the clamps, set 5.0 inches (12.7 cm) apart, of a constant rate of extension tensile testing machine such as an Instron table model tester. A continuously increasing load is applied to the sample at a crosshead speed of 2.0 in/min (5.08 cm/min) until failure. The measurement is given in percentage of stretch prior to failure. The test generally follows ASTM D 5035-95.

Bond strength is measured according to a test that generally follows the method of ASTM D2724-87, which is hereby incorporated by reference. The test was performed used a constant rate of extension tensile testing machine such as an Instron table model tester. A 2.54 cm (1.0 in) by 20.32 cm (8.0 in) sample is delaminated approximately 3.18 cm (1.25 in) by initiating a separation between the fibrous web and the moisture vapor permeable film. The separated sample faces are mounted in the clamps of the tester which are set 5.08 cm (2.0 in) apart. The tester is started and run at a crosshead speed of 25.4 cm/min (10.0 in/min). The computer starts picking up readings after the slack is removed, nominally a 5 gram pre-load. The sample is delaminated for about 12.7 cm (5 in) during which sufficient readings are taken to provide a representative average of the data. The average bond strength is given in g/inch. For samples that are peeled the entire 12.7 cm (5 inches) the average bond strength is considered to be the bond strength. For wet testing, samples were immersed in water for 5 minutes, and excess water was removed by paper towels before testing.

Hydrostatic head was measured according to ISO 811, which measures the resistance to water penetration on a 7 in x 7 in (18 cm x 18 cm) test sample. Water pressure is applied to the fabric side of the test specimen until the sample is penetrated by water at three places. The hydrostatic pressure is measured in inches and converted to SI units and is reported in cm of water. The equipment used to measure hydrostatic head is made by Aspull Engineering Ltd, England.

Moisture Vapor Transmission Rate (MVTR) is reported in g/m²/24 hrs and was measured with a Lyssy Instrument using test method TAPPI T-523.

Viral Barrier properties were measured according to ASTM F1671. ASTM F1671 is a standard test method for measuring the resistance of materials used in protective clothing to penetration by blood-borne pathogens. According to this method, three samples of a sheet material being tested are challenged with 10⁸ Phi-X174 bacteriophage, similar in size to the Hepatitis C virus (0.028 micrometers) and with a surface tension adjusted to 0.042 N/m, at a pressure differential of 2 psi (13.8 kPa) for a 24 hour period. Penetration of the sample by viable viruses is determined using an assay procedure. The test results are reported in units of Plaque Forming Units per milliliter PFU/ml. A sample fails if any viral penetration is detected through any of the samples. A sample passes if zero PFU/ml were detected after the 24 hour test period.

A positive and negative control is run with each sample set. The positive control was a microporous membrane with a pore size of 0.04 micrometers which passed 600 PFU/ml. The negative control was a sheet of Mylar® film, which passed 0 PFU/ml.

Handle-o-meter was measured according to INDA Standard Test (IST) 90.3. IST 90.3 is a standard test method for measuring the resistance of a 10.16 by 10.16 cm (4-inch by 4-inch) test fabric to fold, which is an indicator for softness. The handle-o-meter was reported in gram force. For each direction, machine (MD) and cross (CD), test results from both surfaces were averaged.

Mullen burst was measured according to ASTM D-3786. ASTM D-3786 is a standard test method for measuring the resistance of a 10.16 by 12.7 cm (4-inch by 5-inch) test sample to bursting using a hydraulic diaphragm tester. The Mullen burst was reported in kPa (psi). For wet testing, samples were immersed in water for 10 seconds and excess water was removed by paper towels before testing.

Fiber size was measured by capturing a test sample image by a camera and evaluating the picture by an image analysis program. The distance across fiber longitudinal edges was measured as a fiber diameter and the averaged fiber diameter was reported in micrometers.

Bending Length was measured according to ASTM D-5732. ASTM D-5732 is a standard test method of measuring the stiffness of a fabric. It is determined as the length that a test strip bends under its own weight. The length of the overhang was reported in cm.

Alcohol Repellency was measured by INDA IST 80.8. IST 80.8 is a standard test method for measuring the resistance of nonwoven fabrics to penetration by aqueous isopropanol solutions. The alcohol repellency was reported in ratings based upon alcohol concentrations. The highest number of test solution that did not penetrate the test specimen within five minutes was recorded.

Electrostatic Decay was measured by INDA IST 40.2. IST 40.2 is a standard test method for measuring the time required to dissipate a charge from the surface of a test specimen. The test sample was charged by positive or negative high voltage (5000V) and time for the charge dissipation was measured. The electrostatic decay was reported in seconds.

### Example 1

The powder-bonded nonwoven layer used in this example was a 100% polyester powder-bonded nonwoven web having a basis weight of 0.5 oz/yd² (17 g/m²) (obtained from HDK Company, Greenville, South Carolina). The powder-bonded nonwoven layer was formed from DuPont Dacron® Type 54W polyester staple fibers 3.81 cm (1.5 inches) in length and having a denier per filament of 1.5 (1.7 dtex). A copolyester powder adhesive is used at a loading of 18 percent by weight based on the total weight of the powder adhesive and staple fibers in the nonwoven web.

The powder-bonded nonwoven web was extrusion coated with a polymer film layer of Hytrel® G4778 copolyether ester (melting point 208°C, vicat softening temperature of 175°C, a shore hardness of 47D, and a water absorption of 2.3%, sold by DuPont) using the process shown in Figure 2. The Hytrel® polymer was fed in pellet form into a screw extruder and melted at a temperature of 440°F (227°C) and fed to a 30 mil (762 µm) by 102 cm die opening in a heated die block maintained at 232 °C. The powder-bonded nonwoven substrate was spaced about 12 inches (30.5 cm) below the opening of the die. The film was extruded at a rate of 116 ft/min (35.4 m/min) to obtain a film thickness of 0.55 mil (14 micrometers). The film was joined to the fibrous powder-bonded nonwoven substrate by passing the coated web through a pair of nip rolls with a nip roll pressure of 689 kPa (100 psi) to form a composite laminated sheet. The nip roll that faced the polymer melt was a silicone rubber roll having a matte finish. The quench bath temperature was maintained at a temperature of 80°F (27°C).

### Comparative Examples A and B

Comparative Examples A and B, as indicated in Table 1 below, were formed in a manner similar to the composite laminates of Example 1, except that a spunbonded polypropylene was used as the nonwoven layer, in place of the 100% polyester powder-bonded nonwoven web. In the Comparative Examples A and B, the basis weight of the nonwoven spunbonded polypropylene fabric webs was varied between 16.96 and 25.43 g/m² (0.5 and 0.75 oz/yd²). The nonwoven webs of Comparative Examples A and B were bonded to the Hytrel® G4778 copolyether ester polymer film layer at a temperature of 470°F (243°C) in order to partially melt the polypropylene and provide adequate bonding to the Hytrel® film layer. It was necessary to add a compatibilizer (13wt% Bynel 50E631 ) to the Hytrel ® film layer to improve its bond strength to the polyolefin nonwoven webs of Comparative Examples A and B. This compatibilizer was not used in Example 1.

### Comparative Example C

A 1 mil thick Hytrel® G4778 copolyether ester polymer film layer was extruded onto a 100% spunlaced polyester nonwoven web at a melt temperature of 450-460°F (232-238°C) at a nip pressure of 689 kPa (100 psi) at 91.4 m/min (300 ft/min) to produce a polyester film/polyester nonwoven composite for comparison to Example 1.

The comparisons between the characteristics of Example 1 and the comparative examples are set forth in Table 1, below.

**TABLE 1**

| Example # | Nonwoven Basis Weight/gm⁻² (oz/yd2) | Film Thickness/mm (mil) | MVTR (g/m²/day) | HH/cm (inch) | Viral Barrier |
|---|---|---|---|---|---|
| Ex. 1 | 16.96 (0.5) | 0.013 (0.55) | 999 | >399 (>157) | Compliant |
| C.Ex. A | 16.96 (0.5) | 0.029 (1.15) | 1133 | 351 (138) | Failed |
| C.Ex. B | 25.43 (0.75) | 0.029 (1.15) | 1254 | 287 (113) | Failed |
| C. Ex. C | 40.69 (1.2) | 0.025 (1) | n/a | n/a | Failed |

The data in Table 1 indicate that the composite laminates utilizing powder-bonded nonwoven layers according to the present invention demonstrate unexpectedly good viral barrier properties relative to conventional composite laminates, even when the polymer film layer is less than one-half the thickness of the conventional laminates. Likewise, the Example 1 laminate of the present invention has better liquid barrier property (hydrohead), while maintaining roughly equivalent MVTR to Comparative Examples A and B. Comparative Example C failed the viral barrier test, which indicates that even when the composite laminate has a 100% polyester nonwoven layer, unless the nonwoven layer is a powder-bonded layer, it has insufficient viral barrier properties.

### Example 2

The powder-bonded nonwoven layer (obtained from HDK Company, Greenville, South Carolina) used in this example was same as Example 1 but the powder loading was increased to 20 percent by weight. The increase in the powder loading may improve surface abrasion resistance of the nonwoven and bonding strength of Hytrel film to the nonwoven.

The powder-bonded polyester nonwoven web was extrusion-coated with a polymer film of Hytrel® 8206 using the process shown in Figure 2 at Scapa Tapes, North America (located in Liverpool, NY). Hytrel® 8206, copolyether ester (melting point 200°C, vicat softening temperature of 151°C, a shore hardness of 45D, and a water absorption of 30%, sold by DuPont) was utilized. The film had a three layer structure from a coextrusion process by two extruders. All three layers consisted of Hytrel® 8206. In addition, 6 % of a blue color concentrate compounded with Hytrel® 4056 was added into the extrusion hopper.

The Hytrel® polymer and additive pellets were dried and fed into the hopper and melted at 444 °F (229 °C). The barrel zone temperatures for the extruders were in the range of 455 (235) to 470 °F (243 °C). The heated die block maintained its temperature in the range of 455 (235) to 496 °F (258 °C). The film was extruded to obtain a film thickness of 0.6 mil (15 micrometers). A nip roll pressure was 276 kPa (40 psi) and the chill roll temperature was 55 °F (13 °C).

The physical properties of the bilaminate from Examples 1 and 2 are in Table 2.

Hytrel® 8206 was selected for Example 2 in order to reduce the noise level of the bilaminate, and also to increase the MVTR of the resulting bilaminate. Hytrel® 8206 has more ether than ester parts, resulting in a softer sheet material and a higher MVTR than sheet material made using Hytrel® G4778. Hytrel® G4778 has been found to provide greater strength and lower water absorbency than Hytrel® 8206.

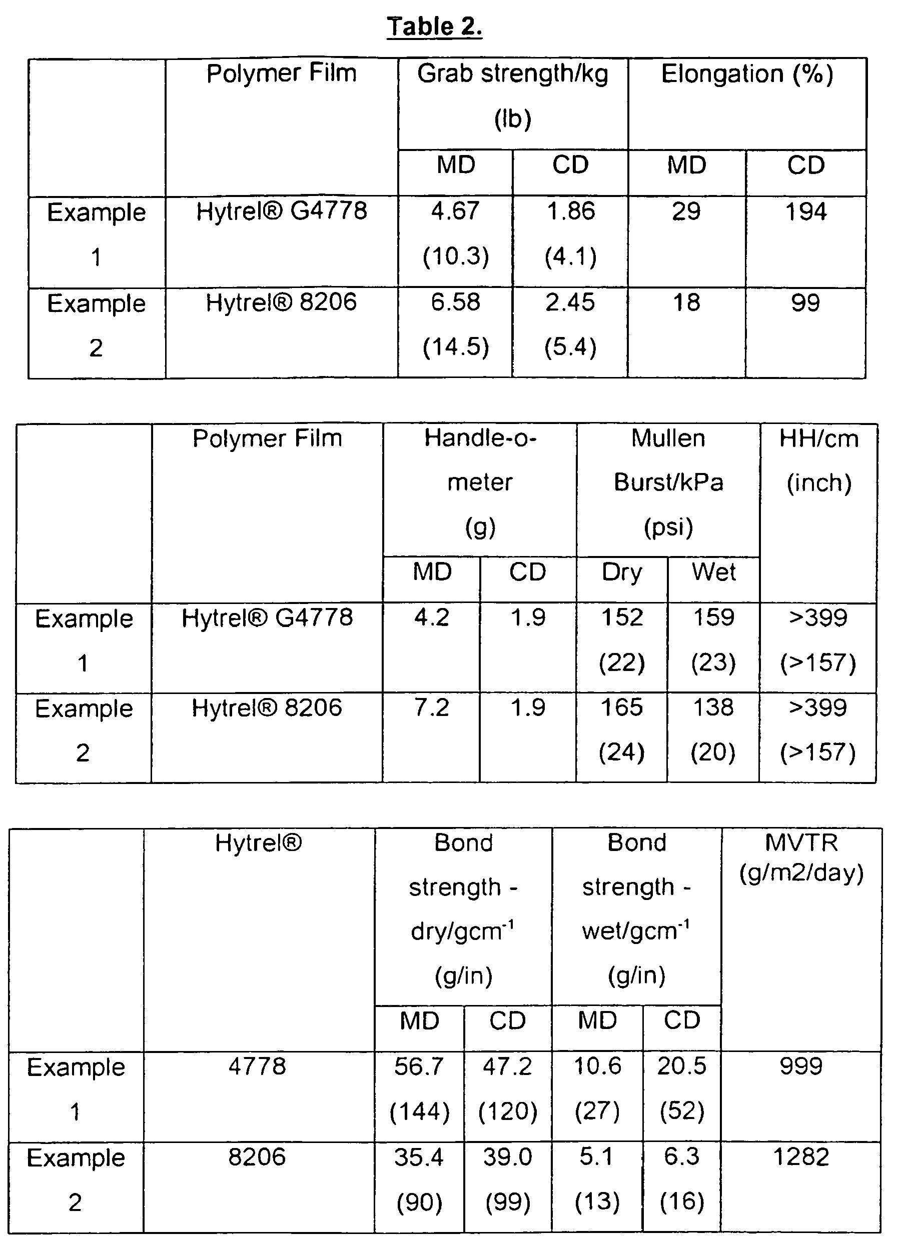

### Examples 3-26

In these examples, a second layer of a nonwoven fabric was added onto the two-layered composites from Examples 1 and 2 to form trilaminate structures. The bilaminates from the aforementioned extrusion coating process were laminated with a spunbonded polypropylene nonwoven web having a basis weight of 20.3 g/m² (0.6 oz/yd²)obtained from BBA Nonwovens (located at Washougal, WA). For alcohol repellency and antistatic properties, the spunbonded polypropylene nonwoven was topically treated with a fluorochemical and an antistatic agent. The physical properties of the spunbonded nonwoven are in Table 3.

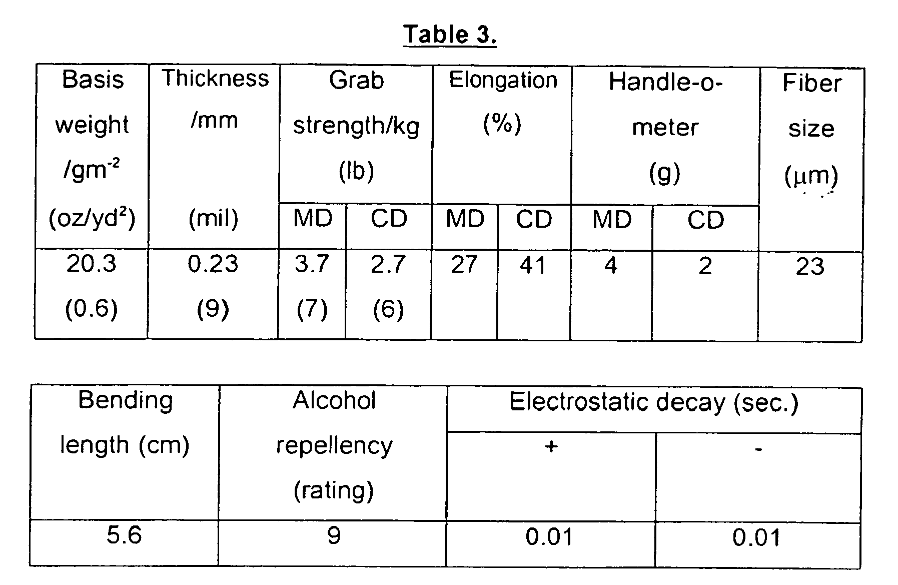

The above spunbonded polypropylene nonwoven was laminated with the composite sheet materials from Examples 1 and 2 by using a meltblown adhesive lamination technology. Three different types of hot melt adhesives were evaluated with add-on levels from 1 to 4 g/m². The adhesives, based on polystyrene-polyisoprene-polystyrene linear block copolymers, H2465, H2485 and H2755, were provided by Bostik Findley (located in Wauwatosa, WI).

The hot melt adhesives were fed and melted in a hopper and transferred by an insulated hose into a 35.6 cm (14-inch) wide ITW Dynatec^{™} line at ITW Dynatec, An Illinois Tool Works Company located in Hendersonville, TN) equipped with nozzles having 7 holes/inch (2,8 holes/cm) tips. The adhesive was melt-blown onto the Hytrel® side of the two-layered composites with a line speed of 183 m/min (600 ft/min), an air pressure of 124 kPa (18 psi) and a nip pressure of 414 kPa (60 psi). The distance from the nozzle head to the bilaminate substrate was 2.54 cm (one inch). The pump speeds for add-on levels of 1, 2, 3 and 4 g/m² were 15, 32, 47 and 59 rpm, respectively. The hopper, hose, head and air temperatures were selected based on the melt viscosity properties of the hot melt adhesives. For H2465, hopper, hose, head and air temperatures were 149, 163, 163 and 177°C (300, 325, 325 and 350 °F), respectively. For H2485, the processing temperatures were 177,191, 204 and 216°C (350, 375, 400 and 420 °F). For H2755, the temperatures were 191, 204, 216 and 216°C (375, 400, 420 and 420 °F). The physical properties of the three layered composites are in Table 4.

**Table 4.**

| **Example No.** | **Adhesive type** | **Bilaminate** | **Add-on (grams)** | **Bond strength - dry/gcm**^{**-1**} **(g/in)** | | **Bond strength - wet/gcm**^{**-1**} **(g/in)** | |
|---|---|---|---|---|---|---|---|
| | | | | MD | CD | MD | CD |
| 3 | H2465 | Example 1 | 1 | 5. 9 | 7.5 | 1.2 | 1.2 |
| | | | | (15) | (19) | (3) | (3) |
| 4 | H2465 | Example 1 | 2 | 30.3 | 15.0 | 3.5 | 2.8 |
| | | | | (77) | (38) | (9) | (7) |
| 5 | H2465 | Example 1 | 3 | 108.7 | 42.1 | 9.1 | 1.2 |
| | | | | (276) | (107) | (23) | (3) |
| 6 | H2465 | Example 1 | 4 | 50.8 | 56.7 | 6.7 | 4.7 |
| | | | | (129) | (144) | (17) | (12) |
| 7 | H2465 | Example 2 | 1 | 3.9 | 4.3 | 0 | 0 |
| | | | | (10) | (11) | | |
| 8 | H2465 | Example 2 | 2 | 17.3 | 18.9 | 0 | 0 |
| | | | | (44) | (48) | | |
| 9 | H2465 | Example 2 | 3 | 23.2 | 22.8 | 0 | 0 |
| | | | | (59) | (58) | | |
| 10 | H2465 | Example 2 | 4 | 42.1 | 48.0 | 0 | 0 |
| | | | | (107) | (122) | | |
| 11 | H2485 | Example 1 | 1 | 10.6 | 4.7 | 2.0 | 3.1 |
| | | | | (27) | (12) | (5) | (8) |
| 12 | H2485 | Example 1 | 2 | 16.1 | 28.0 | 5.9 | 10.2 |
| | | | | (41) | (71) | (15) | (26) |
| 13 | H2485 | Example 1 | 3 | 89.0 | 66.5 | 8.3 | 13.4 |
| | | | | (226) | (169) | (21) | (34) |
| 14 | H2485 | Example 1 | 4 | 61.8 | 58.3 | 11.4 | 13.0 |
| | | | | (157) | (148) | (29) | (33) |
| 15 | H2485 | Example 2 | 1 | 2.4 | 1.6 | 0 | 0 |
| | | | | (6) | (4) | | |
| 16 | H2485 | Example 2 | 2 | 12.2 | 6.3 | 0 | 0 |
| | | | | (31) | (16) | | |
| 17 | H2485 | Example 2 | 3 | 41.3 | 24.8 | 0 | 0 |
| | | | | (105) | (63) | | |
| 18 | H2485 | Example 2 | 4 | 46.5 | 19.7 | 0 | 0 |
| | | | | (118) | (50) | | |
| 19 | H2755 | Example 1 | 1 | 8.7 | 8.3 | 9.1 | 3.5 |
| | | | | (22) | (21) | (23) | (9) |
| 20 | H2755 | Example 1 | 2 | 35.8 | 4.3 | 33.1 | 25.6 |
| | | | | (91) | (11) | (84) | (65) |
| 21 | H2755 | Example 1 | 3 | 92.9 | 55.1 | 76.8 | 30.7 |
| | | | | (236) | (140) | (195) | (78) |
| 22 | H2755 | Example 1 | 4 | 113.0 | 55.9 | 99.6 | 93.3 |
| | | | | (287) | (142) | (253) | (237) |
| 23 | H2755 | Example 2 | 1 | 7.5 | 7.5 | 0 | 0 |
| | | | | (19) | (19) | | |
| 24 | H2755 | Example 2 | 2 | 44.5 | 41.3 | 0 | 0 |
| | | | | (113) | (105) | | |
| 25 | H2755 | Example 2 | 3 | 74.8 | 57.1 | 0 | 0 |
| | | | | (190) | (145) | | |
| 26 | H2755 | Example 2 | 4 | 46.1 | 84.6 | 0 | 0 |
| | | | | (117) | (215) | | |

The general trend from the bond strength tests indicates that the increase in the add-on levels of hot melt adhesive resulted in the increase in the bond strengths of the three-layered laminates. The results show that the trilaminates fabricated by using H2755 had higher bond strengths both in dry and wet. The wet strengths from H2755 were significantly greater than those from other types of adhesives. The trilaminates including Example 2 had no wet strength due to the high water absorption (30%) of Hytrel® 8206. In the wet condition, the volume of Hytrel® 8026 film was increased and the trilaminates were easily delaminated. Therefore, high wet strengths were obtained from the trilaminates having Example 1 as the bilaminate and H2755 as the hot melt adhesive.

The skilled artisan will recognize that the composite laminates according to the present invention can have use in a number of other possible applications in which moisture vapor permeability in combination with liquid impermeability is important, such as protective covers for automobiles, crops, housewrap and roofliner. The viral barrier characteristics of the present invention also lend it to use in protective apparel other than medical garments, such as protective apparel to prevent particulate penetration, such as asbestos; protection from other biological agents such as bacteria; protection from harmful liquid penetration, such as harsh chemicals; and cleanroom garments. Likewise, the composite laminates of the present invention can find use in personal care articles, such as diapers, sanitary napkins and the like. The vapor permeability of the present invention lends it to use in steam sterilization wraps for wrapping surgical instruments and supplies for sterilization.

## Claims

1. A moisture vapor permeable, substantially liquid impermeable composite sheet material having a hydrohead measured according to ISO-811 of at least 50 cm, and a moisture vapor permeability of at least 600g/m²/24 hr, comprising:
a moisture vapor permeable non-porous polymeric film having a first side and a second side,
a first nonwoven layer comprising a moisture vapor permeable powder-bonded nonwoven layer, said powder-bonded layer comprising a nonwoven web of fibers, wherein greater than 95 weight percent of the fibers in the nonwoven web are compatible with said polymeric film, said first nonwoven layer being adhered to the first side of the polymeric film by extrusion of said film onto said first nonwoven layer, and
a second moisture vapor permeable nonwoven layer adhered to the second side of the film, wherein said second nonwoven layer is not a powder-bonded layer.

2. The moisture vapor permeable, substantially liquid impermeable composite sheet material according to claim 1, wherein said second nonwoven layer is selected from the group consisting of spunbond, hydroentangled, stitchbonded, and flash spun plexifilamentary nonwoven fabrics.

3. The moisture vapor permeable, substantially liquid impermeable composite sheet material according to claim 1, wherein said non-porous polymeric film comprises a polymer selected from the group consisting of a block polyether ester copolymer, a polyetheramide copolymer, a polyurethane copolymer, a poly(etherimide) ester copolymer, polyvinyl alcohols, or a combination thereof.

4. The moisture vapor permeable, substantially liquid impermeable composite sheet material according to claim 1, wherein said first nonwoven layer is selected from the group consisting of poly(ethylene terephthalate), poly(1,3-propylene terephthalate) and copolymers thereof.

5. The moisture vapor permeable, substantially liquid impermeable composite sheet material according to claim 1, wherein said composite sheet has a bond strength of at least 19.7 grams/cm (50 grams/inch) between the second nonwoven layer and the film when said composite sheet is either wet or dry.

6. The moisture vapor permeable, substantially liquid impermeable composite sheet material according to claim 1, which passes the viral barrier test according to ASTM F-1671.

7. The moisture vapor permeable, substantially liquid impermeable composite sheet material according to claim 1, wherein said polymeric film is no more than 25 micrometers thick.

8. The moisture vapor permeable, substantially liquid impermeable composite sheet material according to claim 1, wherein said polymeric film is a moisture vapor permeable non-porous block copolyether ester film.

9. A garment made from the composite sheet material of claim 1, in which the first nonwoven layer forms the outer surface.

10. A protective cover for automobiles made from the composite sheet material of claim 1.

11. A protective cover for crops made from the composite sheet material of claim 1.

12. Housewrap made from the composite sheet material of claim 1.

13. A roofliner made from the composite sheet material of claim 1.

14. A diaper made from the composite sheet material of claim 1.

15. A sanitary napkin made from the composite sheet material of claim 1.

16. A cleanroom garment made from the composite sheet material of claim 1.

17. A steam sterilization wrap made from the composite sheet material of claim 1.

## Patentansprüche

1. Bahnförmiges Verbundmaterial, welches für Wasserdampf durchlässig und für Flüssigkeit hingegen im Wesentlichen undurchlässig ist und welches eine gemäß ISO-811 gemessene hydrostatische Druckhöhenzahl (hydrohead) von mindestens 50 cm aufweist und eine Durchlässigkeit für Wasserdampf von mindestens 600 g/m²/24 Std. und welches umfasst:
einen für Wasserdampf durchlässigen, nicht porösen, polymeren Film mit einer ersten Seite und mit einer zweiten Seite,
eine erste nicht gewebte Schicht, welche eine für Wasserdampf durchlässige sowie mit Pulver gebundene und nicht gewebte Schicht umfasst, wobei die mit Pulver gebundene Schicht ein nicht gewebtes Vlies von Fasern darstellt, bei welchem mehr als 95 Gewichtsprozent der Fasern in dem nicht gewebten Vliesstoff verträglich mit dem polymeren Film sind, wobei die erste nicht gewebte Schicht an der ersten Seite des polymeren Films anhaftet infolge einer Extrusion des Films auf die erste nicht gewebte Schicht, und
eine zweite für Wasserdampf durchlässige, nicht gewebte Schicht, welche an der zweiten Seite des Films anhaftet, wobei die zweite nicht gewebte Schicht keine mit Pulver gebundene Schicht darstellt.

2. Bahnförmiges Verbundmaterial, welches für Wasserdampf durchlässig und für Flüssigkeit hingegen im Wesentlichen undurchlässig ist, gemäß Anspruch 1, bei welchem die zweite nicht gewebte Schicht ausgewählt ist aus der Gruppe bestehend aus mit Wasserstrahlen verfestigten Vliesstoffen, aus durch Nähwirken verfestigten Vliesstoffen und aus flash gesponnenen, plexifaserförmigen, nicht gewebten Vliesstoffen.

3. Bahnförmiges Verbundmaterial welches für Wasserdampf durchlässig und für Flüssigkeit hingegen im Wesentlichen undurchlässig ist, gemäß Anspruch 1, bei welchem der nicht poröse, polymere Film ein Polymer umfasst, welches ausgewählt ist aus der Gruppe bestehend aus einem Blockpolyetherestercopolymer, einem Polyetheramidcopolymer, einem Polyurethancopolymer, einem Poly(etherimid)estercopolymer, Polyvinylalkoholen oder aus einer Kombination derselben.

4. Bahnförmiges Verbundmaterial, welches für Wasserdampf durchlässig und für Flüssigkeit hingegen im Wesentlichen undurchlässig ist, gemäß Anspruch 1, bei welchem die erste nicht gewebte Schicht ausgewählt ist aus der Gruppe bestehend aus Poly(ethylenterephthalat), Poly(1,3-propylenterephthalat) und aus Copolymeren derselben.

5. Bahnförmiges Verbundmaterial, welches für Wasserdampf durchlässig und für Flüssigkeit hingegen im Wesentlichen undurchlässig ist, gemäß Anspruch 1, bei welchem das bahnförmige Verbundmaterial eine Verbundfestigkeit von mindestens 19,7 Gramm/cm (50 Gramm/Inch) zwischen der zweiten nicht gewebten Schicht und dem Film aufweist, wenn das bahnförmige Verbundmaterial entweder nass oder trocken ist.

6. Bahnförmiges Verbundmaterial, welches für Wasserdampf durchlässig und für Flüssigkeit hingegen im Wesentlichen undurchlässig ist, gemäß Anspruch 1, welches den viralen Barrieretest gemäß ASTM F-1671 besteht.

7. Bahnförmiges Verbundmaterial, welches für Wasserdampf durchlässig und für Flüssigkeit hingegen im Wesentlichen undurchlässig ist, gemäß Anspruch 1, bei welchem der polymere Film nicht dicker als 25 Mikrometer ist.

8. Bahnförmiges Verbundmaterial, welches für Wasserdampf durchlässig und für Flüssigkeit hingegen im Wesentlichen undurchlässig ist, gemäß Anspruch 1, bei welchem der polymere Film aus einem für Wasserdampf durchlässigen, nicht porösen Blockcopolyetheresterfilm besteht.

9. Kleidungsstück, das aus dem bahnförmigen Verbundmaterial gemäß Anspruch 1 hergestellt, bei welchem die erste nicht gewebte Schicht die äußere Oberfläche bildet.

10. Schutzabdeckung für Automobile, die aus dem bahnförmigen Verbundmaterial gemäß Anspruch 1 hergestellt sind.

11. Schutzabdeckung für Ernten, die aus dem bahnförmigen Verbundmaterial gemäß Anspruch 1 hergestellt sind.

12. Gebäudeverkleidung, die aus dem bahnförmigen Verbundmaterial gemäß Anspruch 1 hergestellt ist.

13. Dachverkleidung, die aus dem bahnförmigen Verbundmaterial gemäß Anspruch 1 hergestellt ist.

14. Windel, die aus dem bahnförmigen Verbundmaterial gemäß Anspruch 1 hergestellt ist.

15. Hygienetücher, die aus dem bahnförmigen Verbundmaterial gemäß Anspruch 1 hergestellt sind.

16. Bekleidung für Reinraum, die aus dem bahnförmigen Verbundmaterial gemäß Anspruch 1 hergestellt ist.

17. Dampfsterilisationshülle, die aus dem bahnförmigen Verbundmaterial gemäß Anspruch 1 hergestellt ist.

## Revendications

1. Matériau composite en feuille perméable à la vapeur d'eau, substantiellement imperméable aux liquides présentant une hauteur d'eau mesurée suivant ISO-811 d'au moins 50 cm et une perméabilité à la vapeur d'eau d'au moins 600 g/m²/24 h, comprenant:
un film polymère non poreux perméable à la vapeur d'eau possédant un premier côté et un deuxième côté,
une première couche non tissée comprenant une couche non tissée perméable à la vapeur d'eau liée par une poudre, ladite couche liée par une poudre comprenant une bande non tissée de fibres, où plus de 95 pour-cent en poids des fibres dans la bande non tissée sont compatibles avec ledit film polymère, ladite première couche non tissée étant adhérée au premier côté du film polymère par extrusion dudit film sur ladite première couche non tissée, et
une deuxième couche non tissée perméable à la vapeur d'eau adhérée sur le deuxième côté du film, où ladite deuxième couche non tissée n'est pas une couche liée par une poudre.

2. Matériau composite en feuille perméable à la vapeur d'eau, substantiellement imperméable aux liquides suivant la revendication 1, dans lequel ladite deuxième couche non tissée est choisie dans le groupe constitué de tissus non tissés à filaments plexiformes filés-liés, hydro-enchevêtrés, liés par piqûre et filés par filage éclair.

3. Matériau composite en feuille perméable à la vapeur d'eau, substantiellement imperméable aux liquides suivant la revendication 1, dans lequel ledit film polymère non poreux comprend un polymère choisi dans le groupe constitué d'un copolymère bloc de polyétherester, d'un copolymère de polyétheramide, d'un copolymère de polyuréthane, d'un copolymère de poly(étherimide)ester, de polyvinylalcools ou d'une combinaison de ceux-ci.

4. Matériau composite en feuille perméable à la vapeur d'eau, substantiellement imperméable aux liquides suivant la revendication 1, dans lequel ladite première couche non tissée est choisie dans le groupe constitué de poly(éthylène téréphtalate), de poly(1,3-propylène téréphtalate) et de copolymères de ceux-ci.

5. Matériau composite en feuille perméable à la vapeur d'eau, substantiellement imperméable aux liquides suivant la revendication 1, dans lequel ladite feuille composite présente une adhérence d'au moins 19,7 grammes/cm (50 grammes/pouce) entre la deuxième couche non tissée et le film lorsque ladite feuille composite est soit humide, soit sèche.

6. Matériau composite en feuille perméable à la vapeur d'eau, substantiellement imperméable aux liquides suivant la revendication 1, qui réussit l'essai de barrière virale suivant ASTM F-1671.

7. Matériau composite en feuille perméable à la vapeur d'eau, substantiellement imperméable aux liquides suivant la revendication 1, dans lequel ledit film polymère n'est pas plus épais que 25 micromètres.

8. Matériau composite en feuille perméable à la vapeur d'eau, substantiellement imperméable aux liquides suivant la revendication 1, dans lequel ledit film polymère est un film de copolyétherester bloc non poreux perméable à la vapeur d'eau.

9. Vêtement fabriqué à partir du matériau composite en feuille suivant la revendication 1, dans lequel la première couche non tissée forme la surface externe.

10. Housse protectrice pour automobiles fabriquée à partir du matériau composite en feuille suivant la revendication 1.

11. Housse protectrice pour récoltes fabriquée à partir du matériau composite en feuille suivant la revendication 1.

12. Revêtement pour bâtiments fabriqué à partir du matériau composite en feuille suivant la revendication 1.

13. Revêtement pour toits fabriqué à partir du matériau composite en feuille suivant la revendication 1.

14. Lange fabriqué à partir du matériau composite en feuille suivant la revendication 1.

15. Serviette hygiénique fabriquée à partir du matériau composite en feuille suivant la revendication 1.

16. Vêtement de salle blanche fabriqué à partir du matériau composite en feuille suivant la revendication 1.

17. Enveloppe de stérilisation à la vapeur fabriquée à partir du matériau composite en feuille suivant la revendication 1.
